# EUROPÄISCHE PATENTSCHRIFT

(11) **EP 2 138 156 B1**
(45) Veröffentlichungstag und Bekanntmachung des Hinweises auf die Patenterteilung: **11.01.2017**
(21) Anmeldenummer: 09160963.6
(22) Anmeldetag: 25.05.2009
(51) Int. Cl.: A61K 8/41, A61Q 5/12, A61K 8/42

(54) **Haarkonditionierende Zubereitung mit einer besonderen Mischung kationischer Konditioniermittel**
Hair conditioner preparation with a special mixture of cationic conditioners
Préparation de conditionnement des cheveux comprenant un mélange particulier d'agents de conditionnement cationiques

(30) Priorität: 27.06.2008 DE 102008030136
(43) Veröffentlichungstag der Anmeldung: 30.12.2009
(73) Patentinhaber: Beiersdorf AG, 20245 Hamburg (DE)
(72) Erfinder: Köhler, Manuela, 22147 Hamburg (DE); Saladin, Sandra, 20144, Hamburg (DE); Demitz, Michael, 22529, Hamburg (DE); Mahadeshwar, Anand, 22529, Hamburg (DE); Wendicke, Silke, 22303, Hamburg (DE)

(56) Entgegenhaltungen:
- EP-A- 1 254 656
- EP-A- 1 878 422
- EP-A- 1 889 602
- EP-B- 1 239 827
- WO-A-2004/100908

## Beschreibung

Haarspülungen werden zur Pflege nach der Reinigung der Haare angewendet. Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar. Es gibt verschiedene Klassen von kationischen Tensiden, die eingesetzt werden um auf die negativ geladenen Keratinfasern aufzuziehen - unter anderem Mono-, Di- oder Trialkylammonium-Verbindungen, Amido Amine, bez. Amidoaminquats sowie auch quaternierte Fettsäuretrialkanolaminestersalze oder "Esterquats." Die Pflegeleistung sowie auch die Sensorik am nassen Haar bei der Anwendung sind stark von der Struktur der Konditionerrohstoffe, sowie auch durch die Abmischung abhängig.

"Gehaltvoll" im Sinne dieser Schrift wird wie folgt festgelegt: Es wird gemessen in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleich bleibenden Druck an der Haarsträhne von oben nach unten. Hierbei wird eine 10er Skala verwendet bei der 10 das höchstmögliche Gleitvermögen darstellt.

"Entwirrbarkeit" im Sinne dieser Schrift ist die Messung wie leicht sich die Haare nach dem Ausspülen des Produkts mit dem Kamm entwirren lassen. Die Haare sind dann entwirrt, wenn der Kamm einmal komplett von oben nach unten durch die Haarsträhne geführt werden kann. Hierbei wird eine 10er Skala verwendet bei der 10 die höchstmögliche Entwirrbarkeit darstellt.
"Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

"Haarstruktur" im Sinne dieser Schrift bedeutet die Beurteilung des Haares durch horizontales Rollen der Haare zwischen Daumen und Zeigefinger/ Mittelfinger einer Hand. Dies wird an verschiedenen Stellen der Strähne beginnend von oben nach unten geprüft. Hierbei wird eine 10er Skala verwendet bei der 10 die höchstmögliche Haarstruktur darstellt.

"Weich geschmeidig" im Sinne dieser Schrift bedeutet das sensorische Empfinden nach Applikation des Produktes im nassen und trockenen Haar durch Expertenpanel.

EP 0786250 B1 offenbart eine wässrige Zubereitung zur avivierenden Faserbehandlung mit 0,01 bis 30 Gew.-% Esterquats und 0,01 bis 30 Gew.-% Alkylamidoaminen.

EP 1254653 B1 offenbart die Verwendung von Mischungen aus Esterquats und Fettsäureamidoaminen zur Herstellung von kosmetischen Zubereitungen.

EP 1037599 B1 offenbart die Verwendung von Detergensgemischen aus Esterquats, erhältlich durch Kondensation von Fettsäuren mit Alkanolaminen und nachfolgende Quaternierung der Alkanolaminoligoester, und Anlagerungsprodukten von Alkylenoxiden an Fettsäureamidoamine zur Herstellung von kosmetischen Zubereitungen

EP 1021502 B1 offenbart Detergensgemische mit oligomeren Esterquats und Anlagerungsprodukten von Alkylenoxiden an Fettsäureamidoaminen.

EP 1254656 B1: offenbart die Mischungen von Esterquats mit quaternierte Fettsäureamidoaminen zur Herstellung von kosmetischen Zubereitungen. Die jeweiligen Zubereitungen werden hinsichtlich ihrer Lagerstabilität untersucht.

EP 1239827 B1: beschreibt transparente Avivagemittel mit speziellen Esterquats mit quaternierte Fettsäureamidoaminen.

Im Stand der Technik fand sich kein Hinweis, wie die Pflegeleistung und/oder die Gleitfähigkeit des nassen Haares verbessert werden konnte.

Diese Gleitfähigkeit ist wichtig für das sensorische Empfinden des Verbrauchers während der Produktapplikation. Je gleitfähiger ein Produkt ist, desto pflegender wird es wahrgenommen. Hierbei wird eine 10er Skala verwendet bei der 10 die höchstmögliche Gleitfähigkeit darstellt.

Es hat sich überraschend gezeigt, dass eine kosmetische Haarbehandlungszubereitung mit kationischen Tensiden, wobei wenigstens 95% der eingesetzten kationischen Tenside Esterquats (EQ) und quaternäre Fettsäuretrialkanolammoniumsalze sind und das Verhältnis quaternäres Fettsäuretrialkanolammoniumsalz zu Esterquat 3/1 bis 1/5 beträgt, wobei flüchtige und/oder nicht flüchtige Silikone enthalten sind und wobei als Esterquat Distearoylethyl Hydroxyethylmonium Methosulfate enthalten ist, zu einer deutlichen Verbesserung der Sensorik während der Applikation der haarkonditionierenden Zubereitung führt. Des Weiteren wurde beobachtet, dass sich die Haarstruktur im nassen und trockenen Haar verbessert. Die Abmischung von Esterquat, nämlich Distearoylethyl Hydroxyethylmonium Methosulfate und quaterniertem Fettsäureamidoamine, nämlich Palmitamidpropyltrimonium Chlorid (Varisoft PATC), führt zu einer hervorragenden Pflegeleistung, wobei Esterquat im Überschuss eingesetzt werden sollte. Desweiteren wurde in einem Experten Sensorikpanel (n=8) festgestellt, dass Abmischungen von quaternierten Fettsäureamidoaminen und Esterquats gegenüber einfachen Fettsäureamidoaminen mit Esterquats überlegen sind. Überraschend gut waren die Verteilbarkeit des Produkts auf dem nassen Haar sowie auch Gehalt, Gleitvermögen und Haarstruktur während des Verteilens, Gehalt, Gleitvermögen und Haarstruktur sofort beim Ausspülen und am Ende des Ausspülens das Gleitvermögen und die Haarstruktur (Referenz Tabelle 2).
Unter der Bezeichnung "Esterquats" werden im allgemeinen quaternierte Fettsäurealkanolamineestersalze verstanden. Es handelt sich hier bevorzugt um Dehyquart F 75, Distearoylethyl Hydroxyethylmonium Methosulfate, von Cognis.

### Dehyquart F 75: Distearoylethyl Hydroxyethylmonium Methosulfate

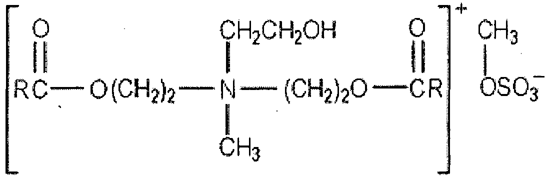

Auch die quaternierten Fettsäureamidoamine sind bekannt, die beispielsweise durch Umsetzung von Fettsäuren mit mehrwertigen Aminen umgesetzt werden, gefolgt von einer nachfolgenden Quaternierung der freien Aminfunktion. Es handelt sich hier bevorzugt um Varisoft PATC, Palmitamidopropyltrimonium Chlorid.

### Varisoft PATC: Palmitamidopropyltrimonium Chlorid

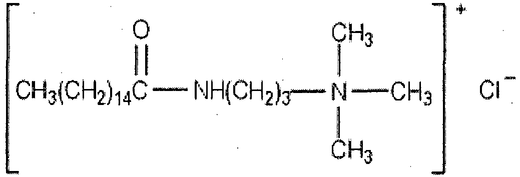

Esterquat (EQ) als einziger Konditionerrohstoff in einer Spülungsformulierung mit Fettalkohol und Dimethicone (DC 200 Fluid 100cst) führte zu einer zwar reichhaltigen Sensorik, jedoch mit Nachteilen bez. Gleitvermögen während der Applikation sowie auch beim Ausspülen hinsichtlich eines stumpfen Haargefühls. Die gleiche Formulierung mit Palmitamidopropyltrimonium Chlorid (PATC) führte zu einem hervorragenden Gleitvermögen, jedoch zeigte die Spülung eine wässrige Sensorik und dünne Viskosität. Laborexpertenpanel Tests zeigten eine überlegene Sensorik und Pflegeleistung für die Kombination EQ-PATC, im Vergleich zu den einzelnen Pflegestoffen. Dabei spielt die Struktur des Fettsäureamidoamins eine wichtige Rolle. PATC hat die beste synergistische Leistung mit EQ gezeigt, im Vergleich zu Stearamidopropyl Dimethylamin, ein Fettsäureamidoamin, der erst in der Spülungsformulierung mit Säure protoniert wird. PATC hat eine CH₂ Gruppe weniger als Stearamidopropyl Dimethylamin und ist schon quaterniert (zusätzliche CH₃ Gruppe).

### Es ist von Vorteil ist es, wenn das Verhältnis quaternäres

Fettsäuretrialkanolammoniumsalz, nämlich Palmitamidopropyltrimonium Chloride, zu Esterquat, nämlich Distearoylethyl Hydroxyethylmonium Methosulfate, 1/2 bis 1/3 beträgt. Das Verhältnis von Esterquat zu PATC ist ganz entscheidend. Weiter von Vorteil ist es, wenn zusätzlich 4 bis 6 Gew.% Glycerin enthalten sind. Weiter von Vorteil ist es, wenn zusätzlich 3 bis 8 Gew.% Cetylstearylalkohol, besonders bevorzugt 4 bis 7 Gew.% Cetylstearylalkohol enthalten sind. Weiter von Vorteil ist es, wenn zusätzlich 0,001 bis 0,08 Gew.% Natriumcitrat oder 0,6 bis 0,8 Gew.% Lactat enthalten sind. Weiter von Vorteil ist es, wenn die Zubereitung nicht waschaktiv ist. Weiter von Vorteil ist es, wenn die Zubereitung frei von anionischen Tensiden ist. Solche Zubereitungen sind bevorzugt vorgesehen für das Ausspülen nach der Anwendung. Damit ist gemeint, dass das Produkt 30 s bis 30 Minuten, bevorzugt 1 bis 5 Minuten im Haar verbleibt. Die Erfindung umfasst auch die Verwendung von Esterquats, nämlich Palmitamidpropyltrimonium Chlorid und quaternären Fettsäuretrialkanolammoniumsalzen, nämlich Distearoylethyl Hydroxyethylmonium Methosulfate, in einer kosmetischen Haarbehandlungszubereitung, vorgesehen für das Ausspülen nach der Anwendung mit kationischen Tensiden wobei wenigstens 95% der eingesetzten kationischen Tenside Esterquats und quaternäre Fettsäuretrialkanolammoniumsalze sind. Die Erfindung umfasst auch die Verwendung einer solchen Zubereitung zur Verbesserung des Haargefühls während der Applikation der Spülung. Oben beschriebene Zubereitungen können auch erfindungsgemäß für den Verbleib im Haar nach der Anwendung vorgesehen sein.
Erfindungsgemäße Zubereitungen können zusätzlich UV- Filter, Polyole, Konservierungsmittel, hydrolysierte Proteine, Pflanzenextrakte, Parfümöle, Antioxidantien enthalten.

### Beispiele

Die Kombination Esterquat/PATC zeigte eine signifikant bessere Pflegeleistung im Vergleich zu Esterquat/Stearamidopropyl Dimethylamin.

**Tabelle 1. Formel 1: AA/EQ (2:2); Formel 2: PATC/EQ (2:2**

| inci | **1** | **2** |
|---|---|---|
| Methylparaben | 0,25 | 0,25 |
| Propylparaben | 0,10 | 0,10 |
| Aqua | 81,84 | 80,52 |
| Citric Acid | 0,15 | 0,15 |
| Cetearyl Alcohol | 5,80 | 5,80 |
| Dimethicone | 1,00 | 1,00 |
| Benzophenone-4 | 0,25 | 0,25 |
| Glycerin | 5,00 | 5,00 |
| Distearoylethyl Hydroxyethylmonium Methosulfate | 2,86 | 2,86 |
| Oryzanol | 0,05 | 0,05 |
| Palmitamidopropyltrimonium Chloride | | 3,32 |
| Stearamidopropyl Dimethylamine | 2,00 | |
| Parfum | 0,70 | 0,70 |

**Tabelle 2. Spülungspanel ; Teilnehmer n=8**

| | **1** | **2** |
|---|---|---|
| Verteilbarkeit | 5,9 | **6,8** |
| Gehalt Verteilen | 5,5 | **6,6** |
| Gleitvermöqen Verteilen | 6,1 | **7,1** |
| Haarstruktur Verteilen | 6,1 | **6,8** |
| Gehalt Sofort | 4,3 | **5,2** |
| Gleitvermögen Sofort | 5,8 | **6,6** |
| Haarstruktur Sofort | 5,9 | **6,6** |
| Ausspülbarkeit | 6,6 | 6,4 |
| Gehalt 40sec | 0,0 | 0,0 |
| Gleitvermögen 40sec | 4,6 | **5,5** |
| Haarstruktur 40sec | 5,0 | **5,5** |
| Entwirrbarkeit | 7,9 | 7,8 |
| Kämmbarkeit | 8,0 | 7,9 |
| Gleitvermögen nass | 5,0 | 5,4 |
| Haastruktur nass | 4,9 | 5,4 |

Das Verhältnis EQ-PATC spielt eine wesentliche Rolle, bez. der Pflegeleistung. In einem Laborexpertenpaneltest (n=5 Experten) wurde die Pflegeleistung sowie auch Sensorik von verschiedenen Abmischungen bewertet.

**Tabelle 3. Verschiedene Abmischungen von PATC/EQ**

| inci | **3** | **4** | **5** | **6** | **7** | **8** | **9** |
|---|---|---|---|---|---|---|---|
| Verhältnis PATC / EQ | **1.5 :1.5** | **2:2** | **1 : 3** | **1 : 2** | **2:1** | **1 : 2** | **1 : 2** |
| Methylparaben | 0,25 | 0,25 | 0,25 | 0,25 | 0,25 | 0,25 | 0,25 |
| Propylparaben | 0,10 | 0,10 | 0,10 | 0,10 | 0,10 | 0,10 | 0,10 |
| Aqua | 83,52 | 80,52 | 81,18 | 82,24 | 81,58 | | |
| Citric Acid | 0,15 | 0,15 | 0,15 | | | 0,01 | 0,01 |
| Cetearyl Alcohol | 4,35 | 5,80 | 5,37 | 5,74 | 6,17 | 2,50 | 3,00 |
| Dimethicone | 1,00 | 1,00 | 1,00 | 1,00 | 1,00 | 0,5 | 1,00 |
| Cyclomethicone | | | | | | 0,5 | 1,0 |
| Benzophenone-4 | 0,25 | 0,25 | 0,25 | 0,25 | 0,25 | | |
| Glycerin | 5,00 | 5,00 | 5,00 | 5,00 | 5,00 | | |
| Sodium Hydroxide | | | | 0,15 | 0,15 | 0,025 | 0,025 |
| Distearoylethyl Hydroxyethylmonium Methosulfate | 2,15 | 2,86 | 4,29 | 2,86 | 1,43 | 2,86 | 2,20 |
| Oryzanol | 0,05 | 0,05 | 0,05 | 0,05 | 0,05 | 0,05 | 0,05 |
| Palmitamidopropyltrimonium Chloride | 2,49 | 3,32 | 1,66 | 1,66 | 3,32 | 1,66 | 1,27 |
| Parfum | 0,70 | 0,70 | 0,70 | 0,70 | 0,70 | 0,70 | 0,70 |

Ein Ranking wurde erstellt hinsichtlich Gehalt, Gleitvermögen, und Pflegeleistung: 6 > 5 > 4 > 7 > 3
Aus diesem Ranking ist zu sehen, dass das beste Verhältnis EQ-PATC (2:1) beträgt sowie auch Vorteile für EQ im Überschuss im Vergleich zu PATC. Das Verhältnis EQ-PATC mit PATC im Überschuss (Formel 7) zeigte ein gutes Gleitvermögen und Nasskämmbarkeit, jedoch keine Produkt reichhaltigkeit während der Applikation. Die vorteilhafte Fettalkohol zu Quaternärekonditionermischungsverhältnisse (FA: Quat) betragen 4.4 : 2. Eine weiteres vorteilhaftes Verhältnis beträgt 3.3 : 2.

Weitere Formelbeispiele:

**Tabelle 4. Formelbeispiele für eine Leave-On Anwendung**

| inci | **10** | **11*** | **12** | **13** |
|---|---|---|---|---|
| Methylparaben | 0,25 | | 0,25 | 0,25 |
| Propylparaben | 0,10 | | 0,10 | 0,10 |
| Glyceryl Stearate | 0,50 | | 0,50 | |
| Aqua | 82,02 | 93,24 | 81,72 | 82,92 |
| Citric Acid | 0,01 | 0,03 | 0,01 | 0,01 |
| Cetearyl Alcohol | 3,20 | | 3,00 | 2,80 |
| Laureth-4 | | 0,50 | | |
| Dimethicone | 0,50 | | | |
| Benzophenone-4 | | 0,10 | | |
| Glycerin | 8,70 | | 8,70 | 8,70 |
| Sodium Hydroxide | 0,03 | 0,03 | 0,03 | 0,03 |
| Aluminum Starch Octenylsuccinate | | 1,00 | | |
| Hydroxypropyl Methylcellulose | 0,25 | | 0,25 | 0,25 |
| Cetrimonium Chloride | | 0,50 | | |
| Distearoylethyl Hydroxyethylmonium Methosulfate | 2,00 | | 2,00 | 2,00 |
| Oryzanol | 0,05 | 0,01 | 0,05 | 0,05 |
| DMDM Hydantoin | | 0,60 | | |
| C12-13 Alkyl Lactate | | 1,00 | | |
| Polyquaternium-37 | | 2,00 | | |
| Cyclomethicone | | | 0,50 | 0,50 |
| Palmitamidopropyltrimonium Chloride | 1,00 | | 1,00 | 1,00 |
| Dimethicone | 0,50 | | 1,00 | 0,50 |
| Parfum | 0,90 | 1,00 | 0,90 | 0,90 |

| | | | | |
|---|---|---|---|---|
| * Nicht erfindungsgemäßes Beispiel | | | | |

## Patentansprüche

1. Kosmetische Haarbehandlungszubereitung mit kationischen Tensiden wobei wenigstens 95% der eingesetzten kationischen Tenside Esterquats und Palmitamidopropyltrimonium Chloride sind und das Verhältnis Palmitamidopropyltrimonium Chloride zu Esterquat 3/1 bis 1/5 ist, **dadurch gekennzeichnet, dass** flüchtige und/oder nicht flüchtige Silikone enthalten sind und **dadurch gekennzeichnet, dass** als Esterquat Distearoylethyl Hydroxyethylmonium Methosulfate enthalten ist.

2. Zubereitung nach Patentanspruch 1, **dadurch gekennzeichnet, dass** das Verhältnis Palmitamidopropyltrimonium Chloride zu Esterquat 2/1 bis 1/3 beträgt.

3. Zubereitung nach einem der vorangehenden Patentansprüche, **dadurch gekennzeichnet, dass** zusätzlich 4 bis 6 Gew.-% Glycerin enthalten sind.

4. Zubereitung nach einem der vorangehenden Patentansprüche, **dadurch gekennzeichnet, dass** zusätzlich 3 bis 8 Gew.-% Cetylstearylalkohol, besonders bevorzugt 4 bis 7 Gew.-% Cetylstearylalkohol enthalten sind.

5. Zubereitung nach einem der vorangehenden Patentansprüche, **dadurch gekennzeichnet, dass** zusätzlich 0,001 bis 0,08 Gew.-% Natriumcitrat oder 0,6 bis 0,8 Gew.-% Lactat enthalten sind.

6. Zubereitung nach einem der vorangehenden Patentansprüche, **dadurch gekennzeichnet, dass** die Zubereitung frei von Oryzanol ist.

7. Zubereitung nach einem der vorangehenden Patentansprüche, **dadurch gekennzeichnet, dass** die Zubereitung nicht waschaktiv ist.

8. Zubereitung nach einem der vorangehenden Patentansprüche, **dadurch gekennzeichnet, dass** die Zubereitung frei von anionischen Tensiden ist.

9. Zubereitung nach einem der vorangehenden Patentansprüche, **dadurch gekennzeichnet, dass** sie vorgesehen für das Ausspülen nach der Anwendung ist.

10. Verwendung einer Zubereitung nach einem der vorangehenden Patentansprüche zur Verbesserung der Gleitfähigkeit des nassen Haares.

11. Zubereitung nach einem der Patentansprüche 1 bis 9, **dadurch gekennzeichnet, dass** sie für den Verbleib im Haar nach der Anwendung vorgesehen ist.

## Claims

1. Cosmetic hair treatment preparation with cationic surfactants wherein at least 95% of the cationic surfactants used are ester quats and palmitamidopropyltrimonium chloride and the ratio of palmitamidopropyltrimonium chloride to ester quat is from 3/1 to 1/5, **characterized in that** volatile and/or non-volatile silicones are present and **characterized in that** distearoylethyl hydroxyethylmonium methosuiphate is present as ester quat.

2. Preparation according to Patent Claim 1, **characterized in that** the ratio of palmitamidopropyltrimonium chloride to ester quat is from 2/1 to 1/3.

3. Preparation according to either of the preceding patent claims, **characterized in that** in addition 4-6% by weight glycerol is present.

4. Preparation according to any of the preceding patent claims, **characterized in that** in addition 3 to 8% by weight cetyl stearyl alcohol, particularly preferably 4 to 7% by weight cetyl stearyl alcohol is present.

5. Preparation according to any of the preceding patent claims, **characterized in that** in addition 0.001 to 0.08% by weight sodium citrate or 0.6 to 0.8% by weight lactate is present.

6. Preparation according to any of the preceding patent claims, **characterized in that** the preparation is free of oryzanol.

7. Preparation according to any of the preceding patent claims, **characterized in that** the preparation is not washing-active.

8. Preparation according to any of the preceding patent claims, **characterized in that** the preparation is free of anionic surfactants.

9. Preparation according to any of the preceding patent claims, **characterized in that** said preparation is intended to be rinsed off after application.

10. Use of a preparation according to any of the preceding patent claims for improving the lubricity of wet hair.

11. Preparation according to any of Patent Claims 1 to 9, **characterized in that** said preparation is intended to remain on the hair after application.

## Revendications

1. Composition cosmétique de traitement des cheveux, présentant des tensioactifs cationiques, au moins 95% des tensioactifs cationiques utilisés étant des esterquats et le chlorure de palmitamidopropyltrimonium et le rapport de chlorure de palmitamidopropyltrimonium à esterquat étant de 3/1 à 1/5, **caractérisée en ce que** des silicones volatils et/ou non volatils sont contenus et **caractérisée en ce que** du méthosulfate de distéaroyléthylhydroxyéthylmonium est contenu comme esterquat.

2. Composition selon la revendication 1, **caractérisée en ce que** le rapport de chlorure de palmitamidopropyltrimonium à esterquat est de 2/1 à 1/3.

3. Composition selon l'une quelconque des revendications précédentes, **caractérisée en ce qu'**elle contient en outre 4 à 6% en poids de glycérol.

4. Composition selon l'une quelconque des revendications précédentes, **caractérisée en ce qu'**elle contient en outre 3 à 8% en poids d'alcool cétylstéarylique, de manière particulièrement préférée 4 à 7% en poids d'alcool cétylstéarylique.

5. Composition selon l'une quelconque des revendications précédentes, **caractérisée en ce qu'**elle contient en outre 0,001 à 0,08% en poids de citrate de sodium ou 0,6 à 0,8% en poids de lactate.

6. Composition selon l'une quelconque des revendications précédentes, **caractérisée en ce que** la composition est exempte d'oryzanol.

7. Composition selon l'une quelconque des revendications précédentes, **caractérisée en ce que** la composition n'est pas active en lavage.

8. Composition selon l'une quelconque des revendications précédentes, **caractérisée en ce que** la composition est exempte de tensioactifs anioniques.

9. Composition selon l'une quelconque des revendications précédentes, **caractérisée en ce qu'**elle est destinée à être éliminée par rinçage après utilisation.

10. Utilisation d'une composition selon l'une quelconque des revendications précédentes pour l'amélioration de l'aptitude au glissement des cheveux humides.

11. Composition selon l'une quelconque des revendications 1 à 9, **caractérisée en ce qu'**elle est destinée à rester sur les cheveux après utilisation.
